# EUROPEAN PATENT SPECIFICATION

(11) **EP 2 950 810 B1**
(45) Date of publication and mention of the grant of the patent: **17.07.2019**
(21) Application number: 14746007.5
(22) Date of filing: 03.02.2014
(51) Int. Cl.: A61L 2/16

(54) **A METHOD OF VIRUS INACTIVATION IN COMPOSITION COMPRISING FACTOR VII**
VERFAHREN ZUR INAKTIVIERUNG VON VIREN IN VERBINDUNG MIT FAKTOR-VII
PROCÉDÉ D'INACTIVATION DE VIRUS DANS UNE COMPOSITION COMPRENANT UN FACTEUR VII

(30) Priority: 31.01.2013 KR 20130011472
(43) Date of publication of application: 09.12.2015
(60) Divisional application: 18160919.9
(73) Proprietor: Hanmi Pharm. Co., Ltd., Hwaseong-si, Gyeonggi-do 445-958 (KR)
(72) Inventor: KIM, Dae Jin, Hwaseong-si Gyeonggi-do 445-764 (KR); LEE, Byung Sun, Seoul 150-103 (KR); KIM, Seung Su, Seoul 137-953 (KR); HWANG, Sang Youn, Hwaseong-si Gyeonggi-do 445-769 (KR); CHOI, In Young, Yongin-si Gyeonggi-do 448-160 (KR); KWON, Se Chang, Seoul 135-506 (KR)
(74) Representative: Mewburn Ellis LLP
(86) International application number: PCT/KR2014/000898
(87) International publication number: WO 2014/119953

(56) References cited:
- EP-A1- 2 275 432
- WO-A1-2010/140140
- WO-A2-2013/051900
- US-A1- 2009 298 760
- US-A1- 2011 052 621
- US-A1- 2012 208 982
- DATABASE GENBANK [Online] 26 December 2006 'SUPEROXIDE DISMUTASE 1, PARTIAL [HOMO SAPIENS]', XP055271319 Retrieved from NCBI Database accession no. ABL96474
- NASIRI ET AL.: 'Preparation of highly purified solvent detergent coagulation factor VII and factor IX concentrates from prothrombin complex (PPSB' MEDICAL JOURNAL OF THE ISLAMIC REPUBLIC OF IRAN vol. 15, no. 2, pages 103 - 108, XP055271321

## Description

### [Technical Field]

The present disclosure relates to a method for inactivating viruses in a composition comprising blood coagulation factor VII, and more particularly, to a method for inactivating viruses including adding a surfactant to a composition comprising blood coagulation factor VII or a derivative thereof, and a method for preparing a virus-inactivated composition comprising blood coagulation factor VII or the derivative thereof.

### [Background Art]

At present, there are an estimated 140 thousand people with hemophilia worldwide, showing an annual increase of 20%. Genetically, hemophilia occurs in one out of every ten thousand, but diagnosis or treatment is made only for approximately 25% of all patients. Based on etiology, hemophilia is largely divided into two types: one is hemophilia A that is caused by a lack of blood coagulation factor VII (Factor VII, FacVII) and accounts for 80% of total hemophilia patients, and the other is hemophilia B that is caused by a lack of blood coagulation factor XI (Factor XI) and accounts for 20% of total hemophilia patients. For the treatment of hemophilia, external administration of blood coagulation factors is given, but this treatment method is problematic in that 10-15% of all hemophilia A patients develop antibodies against the blood coagulation factor, and 1-3% of all hemophilia B patients develop antibodies against the blood coagulation factor.

On the other hand, FacVII, which is a cause of hemophilia A accounting for more than half of hemophilia patients, is an enzyme that is mainly produced in the liver and composed of 406 amino acids, and includes gamma-carboxylation of glutamic acid at position 10, N-glycosylation of asparagines at positions 145 and 322, and O-glycosylation of serines at positions 52 and 60. Further, FacVII has two EGF-like domains and one serine protease domain, and single-chain FacVII is activated through cleavage between arginine at position 152 and isoleucine at position 153 to generate two-chain FacVII a consisting of a light chain and a heavy chain. Since activated FacVII a acts through auxiliary blood clotting mechanism, unlike other blood coagulation factors, antibodies are not produced even though injection of high-dose FacVIIa. Therefore, it can be used for the treatment of hemophilia A patients as well as patients having antibodies against FacVII due to the conventional therapies, and is known as a means of addressing the above described problems.

However, since recombinant production of FacVII using animal cells increases the risk of viral contamination, the process for FacVII production is required to include an elimination process of potential virus. Frequently, a composition that undergoes the inactivation process of viral contaminants has the problem of low FacVII titer, compared to the initial composition prior to inactivation.

WO2010/140140 describes a method for preparing a composition or a concentrate of a prothrombic complex that includes the II, VII, IX and X coagulation factors, wherein said method includes the steps of providing a supernatant of a plasma cryoprecipitate, applying said supernatant on an anion-exchange resin in order to produce an eluate containing said complex and proteins having a high molecular weight, and applying said eluate on a hydroxyapatite column in order to produce a second eluate containing said complex.

US2011/052621 describes the provision of immunogens comprising an antigenic PCSK9 peptide linked to an immunogenic carrier for the prevention, treatment or alleviation of PCSK9-mediated disorders. Also described are methods for production of these medicaments, immunogenic compositions and pharmaceutical compositing thereof and their use in medicine.

WO2013/051900 describes a blood coagulation factor VII derivative, a blood coagulation factor VIia derivative, FacVll and FacVlla conjugates each prepared by linking a polymer capable of extending the blood half-life to the derivative, FacVll and VIia complexes each prepared by linking a carrier to the conjugate, genes encoding the FacVll and FacVlla derivatives, expression vectors comprising the genes, transformants introduced with the expression vectors, a method for preparing the FacVll and FacVlla derivatives using the transformants, a method for preparing the FacVlla conjugate and complex, a FacVlla complex prepared by the method, a pharmaceutical composition for the prevention or treatment of hemophilia comprising the derivative, conjugate, or complex as an active ingredient, and a pharmaceutical composition for blood coagulation comprising the derivative, conjugate, or complex as an active ingredient. Also described is a method for preventing or treating hemophilia or for promoting blood coagulation, comprising administering to a subject a therapeutically effective amount of the composition.

EP2275432 discloses a method for improving the viral safety of liquid Factor VII compositions, in particular those comprising active Factor VII polypeptides (a Factor VIIa polypeptide).

US2009/0298760 discloses FactorVII (FVII) and Factor Vila (FVIIa) albumin linked polypeptides. The disclosure also relates to cDNA sequences coding for human Factor VII and Factor Vila and derivatives genetically fused to a cDNA coding for human serum albumin which may be linked by oligonucleotides which code for intervening peptidic linkers such encoded derivatives, recombinant expression vectors containing such cDNA sequences, host cells transformed with such recombinant expression vectors, and recombinant polypeptides and derivatives.

DATABASE GENBANK entry ABL96474.1 GI:119710570, 26 December 2006 (2006-12-26), discloses the sequence of superoxide dismutase 1, partial [Homo sapiens].

US2012/0208982 describes methods of inactivating a lipid-coat containing virus and proteins essentially free of a lipid-coat containing virus obtained from such methods.

NASIRI et al., MEDICAL JOURNAL OF THE ISLAMIC REPUBLIC OF IRAN, vol. 15, no. 2, pages 103-108, discloses the use of anion-exchange chromatography to purify factor VII and factor IX from prothrombin complex (PPSB), which contains coagulation factors II, VII, IX and X.

Under this background, the present inventors have made many efforts to develop a composition comprising FacVII or a FacVII derivative having the amino acid sequence thereof, in which viruses are effectively inactivated without reducing FacVII titer. As a result, they found that when a surfactant is added to a composition comprising FacVII, contaminated viruses can be inactivated without affecting activity of FacVII or the derivative thereof, thereby completing the present disclosure.

### [Disclosure]

### [Technical Problem]

An object of the present disclosure is to provide a method for inactivating viruses in a composition comprising blood coagulation factor VII (Factor VII, FacVII) or a derivative thereof.

Another object of the present disclosure is to provide a method for preparing a virus-inactivated composition comprising blood coagulation factor VII or the derivative thereof.

### [Technical Solution]

The present invention is as defined in the claims. The present disclosure also describes related matter as set out below.

In one aspect to achieve the above objects, the present disclosure provides a method for inactivating viruses, including the step of adding a surfactant to a composition including blood coagulation factor VII (Factor VII, FacVII) or a derivative thereof.

In one specific embodiment, the content of the activated blood coagulation factor VII or derivative thereof in the composition according to the present disclosure is less than 5%, based on the total blood coagulation factor VII or derivative thereof.

In another specific embodiment, the composition according to the present disclosure is a liquid composition.

In another specific embodiment, the liquid composition according to the present disclosure has pH 5.0 to 8.0.

In another specific embodiment, the liquid composition according to the present disclosure has pH 5.5.

In another specific embodiment, the composition according to the present disclosure has a temperature of 4 to 25°C.

In another specific embodiment, the concentration of the blood coagulation factor VII or the derivative thereof in the composition according to the present disclosure is 0.01 mg/mL to 5.0 mg/mL.

In another specific embodiment, the surfactant used iin the method of the present disclosure is selected from the group consisting of Tween, polysorbate 20, polysorbate 60, polysorbate 80, ploxamer188, Triton X-100, and combinations thereof.

In another specific embodiment, the surfactant is added to the composition of the present disclosure at a final concentration of 0.01 to 1.00 % (w/v).

In another specific embodiment, the surfactant is added to the composition of the present disclosure at a final concentration of 0.1 to 0.5 % (w/v).

In another specific embodiment, the blood coagulation factor VII derivative used in the method of the present disclosure is prepared by linking a peptide linker to the C-terminus of blood coagulation factor VII.

In another specific embodiment, the peptide linker in the blood coagulation factor VII derivative has cysteine as the final amino acid residue of the C-terminus.

In another specific embodiment, the peptide linker in which is linked to the C-termius of the blood coagulation factor VII has one peptide sequence selected from the group consisting of SEQ ID NOs. 3 and 6 to 12.

In another aspect, the present disclosure provides a method for preparing a virus-inactivated composition including blood coagulation factor VII or the derivative thereof comprising adding a surfactant to a composition comprising blood coagulation factor VII or a derivative thereof.

### [Advantageous Effects]

When a virus inactivation method provided by the present disclosure is used, viruses can be inactivated in a solution comprising FacVII and a derivative having the amino acid sequence thereof while maintaining activity of FacVII or FacVII derivative as it is. Therefore, this method can be widely used in the effective production of prophylactic or therapeutic agents for hemophilia.

### [Description of Drawings]

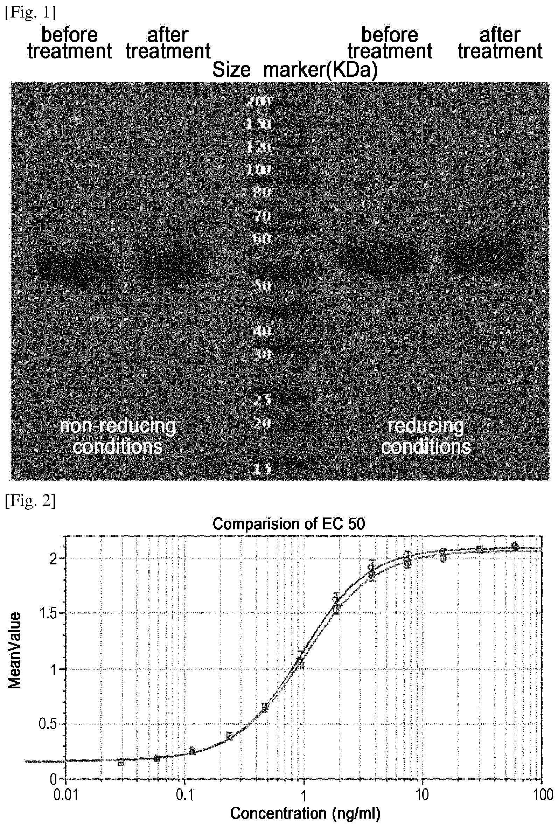

FIG. 1 is an electrophoresis image showing the result of analyzing purity of the FacVII derivative which was expressed in CHO cell line and then purified, before and after virus inactivation by using a surfactant, in which the left side based on the size marker represents the result of analyzing purity of the FacVII derivative under non-reducing condition, and the right side based on the size marker represents the result of analyzing purity of the FacVII derivative under reducing condition.
FIG. 2 is a graph showing the result of analyzing activity of the FacVII derivative which was expressed in CHO cell line and then purified, before and after virus inactivation using a surfactant, in which the circle (○) represents activity of the FacVII derivative before virus inactivation, and the square (□) represents activity of the FacVII derivative after virus inactivation.

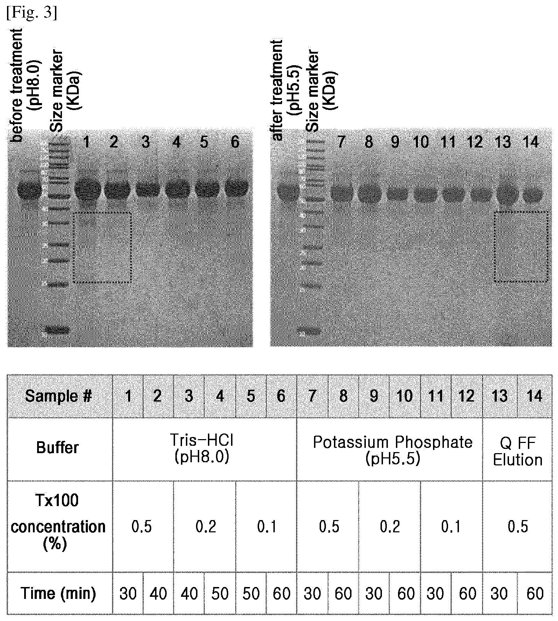

FIG. 3 is an electrophoresis analysis image showing the result of analyzing purity of the FacVII which was expressed in CHO cell line and then purified, before and after virus inactivation by using a surfactant, in which the left image represents the results of analyzing the purity of the FacVII under non-reducing conditions, and the right image represents the results of analyzing the purity of the FacVII under reducing conditions. The dotted box in Fig 3 represents the FacVIIa produced by the virus inactivation process.

### [Mode]

The present disclosure relates to a method for inactivating viruses, comprising adding a surfactant to a composition comprising blood coagulation factor VII (Factor VII, FacVII) or a derivative thereof, or a method for preparing a virus-inactivated composition comprising blood coagulation factor VII or the derivative thereof.

As used herein, the term "blood coagulation factor VII (Factor VII, FacVII)" is, also called proconvertin, one of the factors involved in blood coagulation, and has a size of 48 kDa, and it is encoded by a gene having a size of 12.8 kb, and mainly produced in the liver, and one of vitamin K-dependent plasma proteins. It has been known that FacVII binds to tissue factor (blood coagulation factor III) on the surface of extravascular tissues such as serine protease precursor and smooth muscle cells, tumor tissues, or activated leukocytes, and thus activates blood coagulation factors IX and X, leading to initiation of the extrinsic blood coagulation. In the present disclosure, FacVII may include a native FacVII, chemically modified FacVII derivatives that retain the normal activity of the native FacVII, and variants that have at least 80% amino acid sequence homology, preferably 85%, 90%, or 95% amino acid sequence homology, and more preferably 98% or 99% amino acid sequence homology with the native FacVII while they retain the normal activity of the native FacVII. However, the sequence homology is not limited thereto, as long as they can be applied to the method of the present disclosure.

As used herein, the term "blood coagulation factor VIIa (Factor VIIa, FacVIIa)" means an active form of blood coagulation factor VII. In FacVII activation, the active site of heavy chain is exposed by cleavage between arginine at position 152 and isoleucine at position 153 of FacVII, thereby generating a dimeric form composed of two amino acid sequences (light chain and heavy chain) from FacVII composed of one amino acid sequence. Herein, the arginine residue at position 152 and the isoleucine residue at position 153 are numbered when the first amino acid of the FacVII light chain excluding a signal peptide and a pro-peptide (also called a pre-pro leader sequence) resulting from processing of the initially expressed FacVII polypeptide is regarded as position 1. Since activated FacVIIa acts through auxiliary blood clotting mechanism, unlike other blood coagulation factors, antibodies are not produced even though injection of high-dose FacVIIa.

As used herein, the term "FacVII derivative" means a modified FacVII that is composed of the polypeptide sequence prepared by linking the peptide linker to the C-terminus of FacVII. The FacVII derivative may be in the form that is prepared by linking the peptide linker to the C-terminus of FacVII via a peptide bond, and this form has a fusion protein form. In the present disclosure, the FacVII derivative may form a dimer as described above, when activated by a particular method.

As used herein, the term "peptide linker" is a peptide that is linked to the C-terminus of FacVII and functions to link a carrier capable of extending the blood half-life. In particular, the peptide link may be a peptide linker having cysteine at its C-terminus.

Examples of the peptide linker may include ATKAVC (SEQ ID NO. 3), GGGGSC (SEQ ID NO. 6), amino acid sequence at positions 1 to 149 of SOD1 (SEQ ID NO. 7), amino acid sequence at positions 1 to 149 of mutated SOD1 (SEQ ID NO. 8), amino acid sequence at positions 1 to 90 of SOD1 (SEQ ID NO. 9), amino acid sequence at positions 1 to 90 of mutated SOD1 (SEQ ID NO. 10), amino acid sequence at positions 1 to 25 of SOD1 (SEQ ID NO. 11) and amino acid sequence at positions 1 to 25 of mutated SOD1 (SEQ ID NO. 12), but are not particularly limited thereto.

Further, the FacVII derivative may be a polypeptide (SEQ ID NO. 13) prepared by linking ATKAVC (SEQ ID NO. 3) to the C-terminus of FacVII derivative; a polypeptide (SEQ ID NO. 14) prepared by linking GGGGSC (SEQ ID NO. 6) to the C-terminus of FacVII derivative; a polypeptide (SEQ ID NO. 15) prepared by linking the amino acid sequence at positions 1 to 149 of SOD1 (SEQ ID NO. 7) to the C-terminus of FacVII derivative; a polypeptide (SEQ ID NO. 16) prepared by linking the amino acid sequence at positions 1 to 149 of the mutated SOD1 (SEQ ID NO. 8) to the C-terminus of FacVII derivative; a polypeptide (SEQ ID NO. 17) prepared by linking the amino acid sequence at positions 1 to 90 of the SOD1 (SEQ ID NO. 9) to the C-terminus of FacVII derivative; a polypeptide (SEQ ID NO. 18) prepared by linking the amino acid sequence at positions 1 to 90 of the mutated SOD1 (SEQ ID NO. 10) to the C-terminus of FacVII derivative; a polypeptide (SEQ ID NO. 19) prepared by linking the amino acid sequence at positions 1 to 25 of the SOD1 (SEQ ID NO. 11) to the C-terminus of FacVII derivative; a polypeptide (SEQ ID NO. 20) prepared by linking the amino acid sequence at positions 1 to 25 of the mutated SOD1 (SEQ ID NO. 12) to the C-terminus of FacVII derivative; or the like, but is not particularly limited thereto. Further, with respect to the FacVII of the present disclosure or the derivative thereof, the disclosure of Korean Patent Publication No. 10-2013-0037659 iscited.

According to one embodiment of the present disclosure, when a surfactant is applied to a composition comprising the above described FacVII or derivative thereof, preferably, a composition comprising the activated FacVII or derivative thereof in an amount of less than 5% based on the total FacVII or derivative thereof, viruses included in the composition can be effectively inactivated, impurities generated from FacVIIa processing can be prevented, and a titer of FacVIIa included in the composition can be almost equivalent to that prior to virus inactivation.

As used herein, the term "composition comprising FacVII or the derivative thereof' means a substance including FacVII or the derivative thereof, which is subjected to surfactant treatment. Preferably, the composition may be a liquid composition.

The present inventor has considered the characteristics of FacVII activation to develop a process of virus inactivation in a solution comprising FacVII or the derivative thereof without reducing the titer of FacVIIa included in the virus inactivated composition. FacVII has a single chain structure in which a light chain and heavy chain are interconnected and thus N-terminus of the heavy chain is not exposed, but only the N-terminus of the light chain is exposed. When FacVII is converted to FacVIIa, the active site of the heavy chain is exposed by cleavage between arginine at position 152 and isoleucine at position 153 of FacVII, and the exposed isoleucine at position 153 of FacVII become the amino acid at position 1 of the N-terminus of the heavy chain. Because the N-terminus of the light chain and heavy chain is important for the activity of FacVIIa, the activity of FacVIIa can be reduced compared to that of native FacVIIa when the N-terminus of the light chain or heavy chain is influenced. In addition, as the FacVIIa shows strong serine protease activity, sites important for the activity of FacVIIa can be cleaved during virus inactivation process. Therefore, impurities such as self-cleaved form can be easily generated.

Thus, the present inventor has not applied the virus inactivation process to the composition in which the activated FacVII or derivative thereof is present at a higher ratio than the non-activated FacVII or derivative thereof, but has instead applied the virus inactivation process to the composition in which the non-activated FacVII or derivative thereof is present at a higher ratio than the activated FacVII or derivative thereof, and confirmed that impurities of FacVIIa and FacVIIa titer reduction in the virus inactivated composition can be prevented.

Therefore, when the composition comprising FacVII is treated with a surfactant for inactivation, the composition in which non-activated FacVII or derivative thereof is present at a higher ratio than the activated FacVII or derivative thereof, preferably at a ratio of more than 95%, is treated with the surfactant, which is advantageous in that the titer of the composition is maintained after virus inactivation. Therefore, the FacVIIa or derivative thereof is preferably present in the composition at a ratio of less than 5%.

Further, in order to prevent conversion of FacVII into FacVIIa by unintended activation, the composition is maintained at pH 8.0 or lower, preferably at pH 5.0 to 8.0, and more preferably at pH 5.0 to 5.5 and at a temperature of 25°C or lower, preferably 4 to 25°C, and more preferably 4 to 10°C.

Further, the composition may include FacVII or the derivative thereof at a concentration of 0.01 to 5.0 mg/mL, preferably at a concentration of 0.01 to 1.0 mg/mL.

The composition including FacVII or the derivative thereof may be prepared by obtaining a culture supernatant from a cell producing FacVII or the derivative thereof, for example, a transformant that is transformed with a vector including a polynucleotide encoding FacVII or the derivative thereof or/and a culture thereof, and then purifying the culture supernatant through one or more purification steps such as filtration or/and chromatography (affinity chromatography, hydrophobic interaction chromatography, ion exchange chromatography, size exclusion chromatograph, etc.). Meanwhile, the cell is not particularly limited to a particular type, as long as it is able to produce FacVII or the derivative thereof. Proper cells known in the art may be used, for example, animal cells, plant cells, prokaryotic cells, insect cells or the like may be used. Animal cells are preferred. An example thereof is CHO cells or the like, but is not particularly limited thereto.

As used herein, the term "virus inactivation" means that viral infectivity in the composition including FacVII or the derivative thereof, that is, an ability of the virus to proliferate in the sensitive cells through cell contact, is eliminated or attenuated. In the present disclosure, the virus inactivation is effectively carried out by surfactant treatment.

As used herein, the term "surfactant", also called a surface active agent, is an amphiphilic compound that generally has a hydrophobic group and a hydrophilic group in one molecule, and adsorbs at the interface in a weak solution to reduce the surface tension. Depending on ionization in an aqueous solution, the surfactant is divided into an ionic surfactant and a non-ionic surfactant, and the ionic surfactant is divided into an anionic surfactant having anions as a main component of the surfactant, a cationic surfactant having cations as a main component of the surfactant, and a zwitterionic surfactant having both anions and cations as main components of the surfactant according to ionization in an aqueous solution. The non-ionic surfactant includes polyethylene glycol or the like, the anionic surfactant includes soap, alkylbenzene sulfonic acid salt or the like, the cationic surfactant includes high amine halides, quaternary ammonium salts, alkylpyridinium salts or the like, and the zwitterionic surfactant includes amino acids or the like. In general, the surfactant has cleaning, emulsifying, dispersing, osmosis, and foaming functions. According to the properties thereof, it is widely used as a detergent, a textile treating agent, an emulsifying agent, a flotation agent, a cement foaming agent, a lubricant additive, a sterilizer, a pigment dispersing agent or the like. With respect to the objects of the present disclosure, the surfactant is used as a sterilizer for eliminating viruses, and the proper surfactant may include Tween, polysorbate 20, polysorbate 60, polysorbate 80, ploxamer188, Triton X-100 or the like, and preferably Triton X-100, but is not particularly limited thereto.

For virus inactivation, the surfactant is preferably added in an amount of 0.01 to 1.00% by weight, and preferably 0.01 to 0.5% by weight, based on the total volume of the final composition.

In one embodiment of the present disclosure, the FacVII derivatives were produced in CHO cells introduced with an expression vector including a polynucleotide encoding the polypeptide that is prepared by linking the ATKAVC peptide at the C-terminus of FacVII as the representative FacVII derivative, and a culture supernatant was obtained from the cell culture broth, and then purified to prepare a composition including FacVII (Example 1). In addition, a surfactant Triton X-100 was added to the composition with pH 5.5 at a concentration of 0.2% (w/v), and they were allowed to react at 4°C to carry out virus inactivation (Example 2). Next, it was examined whether purity of the FacVII derivative was changed by the virus inactivation. As a result, no bands of the activated FacVIIa were observed before and after inactivation, and in particular, self-cleaved forms were not observed even after inactivation (Examples 3 and 4). Further, a surfactant Triton X-100 was added to a composition comprising FacVII with pH 5.5 at a concentration of 0.1 to 0.5% (w/v), and they were allowed to react at 4°C to carry out virus inactivation. As a result, no bands of the activated FacVIIa or self-cleaved forms were observed in all of the above samples. In addition, a surfactant Triton X-100 was added to the composition comprising FacVII with pH 8.0 at a concentration of 0.1 to 0.5% (w/v), and they were allowed to react at 4°C to carry out virus inactivation. As a result, no bands of the activated FacVIIa or self-cleaved forms were observed from low concentration conditions, i.e., 0.1 to 0.2 % (w/v) of triton X-100, but the activated FacVIIa was observed when 0.5 % (w/v) of triton X-100 was added to the composition (Examples 5 and 6).

The above results showed that the titers before and after virus inactivation were almost the same when titers of the composition were analyzed and compared before and after virus inactivation. This result indicates that although the method of the present disclosure is applied to the composition including FacVII or the derivative thereof which is known to be easily degraded or modified, viruses are effectively inactivated without damaging the protein structure and reducing the titer.

Hereinafter, the present disclosure will be described in more detail with reference to Examples. However, these Examples are for illustrative purposes only, and the disclosure is not intended to be limited by these Examples.

### Example 1: Preparation of FacVII derivatives

### Example 1-1: Preparation of FacVII gene-containing expression vector

First, human Factor VII gene containing a signal sequence was obtained using a Polymerase Chain Reaction (PCR) technique. For amplification of Factor VII (FacVII) gene, a human fetal liver cDNA library (TAKARA BIO USA) was used as a template, and forward and reverse primers of the following SEQ ID NOs. 1 and 2 were used to perform PCR (95°C 1 minute denaturation; 30 cycles (95°C 30 seconds, 60°C 30 seconds and 68°C 90 seconds); 68°C 5 minutes). At this time, for easy cloning, the recognition site for the restriction enzyme BamHI was inserted into the primer of SEQ ID NO. 1 and the recognition site for the restriction enzyme XhoI was inserted into the primer of SEQ ID NO. 2. Subsequently, a nucleotide sequence of the PCR product of approximately 1.3 kb obtained by PCR was examined.
VII BHISS F: 5'-cccggatccatggtctcccaggccctcaggctcc-3' (SEQ ID NO. 1)
VII XhoIAS R: 5'-gggctcgagctagggaaatggggctcgcagg-3' (SEQ ID NO. 2)

In order to express the obtained PCR product under the control of CMV promoter, it was cloned into an animal cell expression vector pX0GC. The pX0GC vector is an expression vector including one or more CCGCCC repeat sequence-removed DHFR promoter and a DHFR-encoding nucleotide sequence operably linked thereto (Korean Patent No. 880509). Specifically, the PCR product was digested with the restriction enzymes, BamHI and XhoI at 37°C for 2 hours, and applied to a PCR purification kit (Qiagen, USA) so as to obtain the cleaved DNA fragment. The DNA fragment was mixed with the pX0GC vector treated with the restriction enzymes BamHI and Xhol, and cloned using T4 DNA ligase, thereby preparing an expression vector including FacVII gene (pX0GC-FVII).

### Example 1-2: Preparation of recombinant FacVII derivative-expressing vector, pX0GC-F VII ATKAVC

A recombinant FacVII derivative-expressing vector pX0GC-FVII-ATKAVC, which contains a polynucleotide further having a polynucleotide encoding the sequence from 1 to 6 of the SOD1 sequence (ATKAVC, SEQ ID NO. 3) at the 3'-terminus of FacVII gene included in the expression vector pX0GC-F VII prepared in Example 1-1, was prepared. In detail, the expression vector pX0GC-FVII was used as a template, and forward and reverse primers of the following SEQ ID NOs. 4 and 5 were used to perform PCR (95°C 1 minute denaturation; 30 cycles (95°C 60 seconds, 60°C 60 seconds and 68°C 90 seconds); 68°C 5 minutes). At this time, for easy cloning, the recognition site for the restriction enzyme EcoRI was inserted into the primer of SEQ ID NO. 4 and the recognition site for the restriction enzyme XhoI was inserted into the primer of SEQ ID NO. 5. Subsequently, a nucleotide sequence of the PCR product of approximately 1.4 kb obtained by PCR was examined.
FVII EcoRISS F (SEQ ID NO. 4):
   5'-ccggaattcatggccaacgcgttcctggaggagctgcggccgggc-3'
F VII #1XhoIAS R (SEQ ID NO. 5):
   5'-ccgctcgagtcagcacacggccttcgtcgcgggaaatggggctcgcaggaggactcctgggc-3'

In order to express the obtained PCR product under the control of CMV promoter, it was cloned into an animal cell expression vector pX0GC. Specifically, the PCR product was digested with the restriction enzymes, EcoRI and XhoI at 37°C for 2 hours, and applied to a PCR purification kit so as to obtain the cleaved DNA fragment. The DNA fragment was mixed with the pX0GC vector treated with the restriction enzymes EcoRI and Xhol, and cloned using T4 DNA ligase, thereby preparing an expression vector (pX0GC-FVII-ATKAVC) having a FacVII derivative-encoding polynucleotide, which contains a polynucleotide encoding the sequence ATKAVC (SEQ ID NO. 3) from 1 to 6 of the SOD1 sequence linked at the 3'-terminus of FacVII gene.

### Example 1-3: Expression of FacVII derivative (pX0GC-F VII-ATKAVC) in CHO cell line

The expression vector pX0GC-FVII-ATKAVC prepared in Example 1-2 was introduced into DG44/CHO cell line (CHO/dhfr-) that is deficient in DHFR to show incomplete DNA synthesis (Urlaub et al., Somat. Cell. Mol. Genet., 12, 555-566, 1986) to obtain a transformant, and FacVII-ATKAVC derivative was expressed from the transformant.

In detail, the DG44/CHO cell line was cultured to reach 80 to 90% confluence, and the cells were washed with Opti-MEM (Gibco, cat. No. 51985034) three times.

On the other hand, a mixture of 3 ml of Opti-MEM and 5 µg of expression vector pX0GC-FVII-ATKAVC, and a mixture of 3 ml of Opti-MEM and 20µl of lipofectamine (Gibco, cat. no. 18324-012) were left at room temperature for 30 minutes, respectively. Subsequently, the mixtures were mixed, and added to the cultured DG44/CHO cell line. Then, the cells were cultured at 37°C and 5% CO₂ for approximately 18 hours, resulting in introduction of the expression vector pX0GC-FVII-ATKAVC into DG44/CHO cell line. Subsequently, the cultured cells were washed with 10% FBS-supplemented DMEM-F12 (Gibco, cat. no. 11330) three times, and then the medium was added thereto, followed by cultivation for 48 hours. The cultured cells were detached by trypsin treatment, and they were inoculated into MEM-α medium (WELGENE, cat. no. LM008-02)) containing 10% FBS and 1 mg/ml of G418 (Cellgro, cat. no. 61-234 -RG) without selection medium (HT supplement (Hypoxanthine-Thymidine)). Until the transformed cells survived to form colonies, the medium was replaced with the selection medium every 2 or 3 days. Thus, the transformed cells were selected from the separated cells. At this time, in order to increase the expression level of FacVII-ATKAVC derivative in the selected transformed cells, 10 nM MTX (Sigma, cat. no. M8407) was added to the selection medium to gradually increase the concentration, and 2 to 3 weeks later, the content of MTX was increased up to 30 nM.

### Example 1-4: Purification of FacVII-ATKAVC

The transformant prepared in Example 1-3 was cultured to express FacVII-ATKAVC, and the culture broth was centrifuged at 3000 rpm for 5 minutes to obtain a culture supernatant.

The culture supernatant was filtered using a 0.2 µm microfilter, and 0.6 M ammonium sulfate was added thereto. The resultant was applied to a Butyl HP column, and elution was carried out using concentration gradient buffer (20 mM Tris-HCl pH7.5) containing 0.6-0 M ammonium sulfate to obtain an active fraction containing FacVII-ATKAVC.

The buffer condition of the active fraction thus obtained was replaced by 10 mM sodium phosphate buffer (pH 7.0), and then applied to a Heparin HP column. Then, elution was carried out using 0-1.0 M NaCl concentration gradient buffer (10 mM sodium phosphate, pH 7.0) to obtain an active fraction containing FacVII-ATKAVC.

The active fraction was concentrated, and then applied to a Superdex75 column, and then elution was carried out using 150 mM NaCl 20 mM Tris-HCl (pH 7.5) buffer to obtain an active fraction containing FacVII-ATKAVC. The buffer condition of the active fraction thus obtained was replaced by 2 mM benzamidine 20 mM Tris-HCl (pH 7.5), and then applied to a Q FF column. Then, washing (2 mM benzamidine 0.2 M NaCl 20 mM Tris-HCl (pH 8.0) buffer), re-equilibration (2 mM benzamidine 0.1 M NaCl 20 mM Tris-HCl (pH 8.0) buffer) and concentration gradient elution (2 mM benzamidine 25 mM NaCl 35 mM CaCl₂, 20 mM Tris-HCl (pH 8.0) buffer) were carried out to purify FacVII-ATKAVC.

### Example 2: Virus inactivation of solution containing FacVII derivative

The FacVII derivative purified in Example 1 was dissolved in a final buffer solution (10 mM NaAc (pH 5.5), 0.02% Pluronic F-68, 5% Mannitol, 0.01% L-Methionine, 50 mM NaCl) at a final concentration of 0.46 mg/ml through ultrafiltration (Pellicon XL, PLCGC 10 50 cm², Millipore) and diafiltration. For virus inactivation, a surfactant Triton X-100 was added at a concentration of 0.2% (w/v) to a solution containing the FacVII derivative of which purity by size exclusion chromatography was 97% or higher, and slowly stirred at 4°C for 30 minutes. Thereafter, the solution was dissolved in the final buffer solution by size exclusion chromatography.

### Example 3: Purity analysis of virus-inactivated FacVII derivative

Purity of the FacVII derivative that had undergone virus inactivation in Example 2 was analyzed by polyacrylamide gel electrophoresis (SDS-PAGE) (FIG. 1). FIG. 1 is an electrophoresis image showing the result of analyzing purity of the FacVII derivative which was expressed in CHO cell line and then purified, before and after virus inactivation using a surfactant. As shown in FIG. 1, there was no size difference in the molecular weight and purity between the samples before and after virus inactivation, and activated forms such as FacVIIa derivative or self-cleaved forms were not observed.

### Example 4: Titer analyses of virus-inactivated FacVII derivate

Effective concentration 50 (EC₅₀) of the FacVII derivative and the virus-inactivated FacVII derivative was measured using a COASET Chromogenic assay kit (Chromogenix, Italy). The FacVII derivative that had not undergone virus inactivation and the FacVII derivative that had undergone virus inactivation were diluted by 2-fold serial dilution from 60 ng/ml to 0.03 ng/ml using a working buffer included in the kit, and 50 ul thereof was dispensed in each well of a 96-well plate. Thereafter, 50 ul of a combined reagent containing FacX, CaCl₂ and thromboplastin was added to each well, and allowed to react at 37°C for 7 minutes. 50 ul of a substrate was added to each well. Finally, absorbance was measured at 405 nm to analyze EC₅₀ of each FacVII derivative for comparison (FIG. 2 and Table 1). FIG. 2 is a graph showing the result of analyzing activity of the FacVII derivative which was expressed in CHO cell line and then purified, before and after virus inactivation using a surfactant, in which the line with circle (○) represents activity of the FacVII derivative before virus inactivation, and the line with square (□) represents activity of the FacVII derivative after virus inactivation.

**[Table 1]**

| Analysis result of titer of FacVII derivative before and after virus inactivation | | |
|---|---|---|
| | EC₅₀ | Relative activity ratio(vs FacVII derivative) |
| FacVII derivative (0.425 mg/ml) | 0.961 | 100.0 |
| Virus-inactivated FacVII derivative (0.079 mg/ml) | 1.024 | 93.9 |

As shown in FIG. 2 and Table 1, EC₅₀ of the FacVII derivative that had not undergone virus inactivation and the FacVII derivative that had undergone virus inactivation was 0.961 ng/ml and 1.024 ng/ml, respectively, indicating that there was no great difference in EC₅₀ values even after virus inactivation.

### Example 5: Virus inactivation of solution containing FacVII

FacVII was purified by a method similar to the method disclosed in Example 1 and finally eluted by using an elution buffer (2 mM benzamidine 25 mM NaCl 35 mM CaCl₂, 20 mM Tris-HCl (pH 8.0) buffer). Purified FacVII was dissolved in a final buffer solution (10mM Tris (pH 8.0) and 50 mM Photassium phosphate (pH 5.5)) at a final concentration of 1.0 mg/ml through ultrafiltration (Pellicon XL, PLCGC 10 50 cm², Millipore) and diafiltration. For virus inactivation, a surfactant Triton X-100 was added at a concentration of 0.1 to 0.5% (w/v) to a solution containing the FacVII of which purity by SDS-PAGE was 97% or higher, and slowly stirred at 4°C for 30 minutes. Thereafter, the solution was dissolved in the final buffer solution by size exclusion chromatography.

### Example 6: Purity analysis of virus-inactivated FacVII

Purity of the FacVII that had undergone virus inactivation in Example 5 was analyzed by polyacrylamide gel electrophoresis (SDS-PAGE) (FIG. 3). FIG. 3 is an electrophoresis image showing the result of analyzing purity of the FacVII which was expressed in the CHO cell line and then purified, before and after virus inactivation using a surfactant.

As shown in FIG. 3, when triton X-100 was added to a composition comprising FacVII with pH 5.5 at a concentration of 0.1 to 0.5% (w/v), there was no size difference in the molecular weight and purity between the samples before and after virus inactivation, and activated forms such as FacVIIa derivative or self-cleaved forms were not observed. Meanwhile, when a surfactant Triton X-100 was added to the composition comprising FacVII with pH 8.0 at a concentration of 0.1 to 0.5% (w/v), no bands of the activated FacVIIa or self-cleaved form were observed at low concentration conditions, i.e., 0.1 to 0.2 % (w/v) of triton X-100. However, when 0.5 % (w/v) of triton X-100 (high concentration conditions) was added to the composition, activated FacVIIa was observed (dotted box).
## Claims

1. A method for inactivating viruses, comprising adding a surfactant to a composition comprising blood coagulation factor VII derivative,
wherein the blood coagulation factor VII derivative is prepared by linking a peptide linker having cysteine as the final amino acid residue of the C-terminus thereof to the C-terminus of blood coagulation factor VII, and wherein the content of activated blood coagulation factor VII derivative in the composition is less than 5%, based on the total blood coagulation factor VII derivative,
wherein the total blood coagulation factor VII derivative includes both activated and inactivated forms.

2. The method according to claim 1, wherein the composition is a liquid composition.

3. The method according to claim 2, wherein the liquid composition has pH 5.0 to 8.0.

4. The method according to claim 2, wherein the composition has a temperature of 4 to 25°C.

5. The method according to claim 1, wherein the concentration of the blood coagulation factor VII derivative in the composition is 0.01 mg/mL to 5.0 mg/mL.

6. The method according to claim 1, wherein the surfactant is selected from the group consisting of Tween, polysorbate 20, polysorbate 60, polysorbate 80, ploxamer188, Triton X-100, and combinations thereof.

7. The method according to claim 1, wherein the surfactant is added at a final concentration of 0.01 to 1.00 %(w/v).

8. The method according to claim 7, wherein the surfactant is added at a final concentration of 0.1 to 0.5 %(w/v).

9. The method according to claim 1, wherein the peptide linker has one peptide sequence selected from the group consisting of SEQ ID NOs. 3 and 6 to 12.

## Patentansprüche

1. Verfahren zur Inaktivierung von Viren, umfassend das Zusetzen eines Tensids zu einer Zusammensetzung, die ein Derivat des Blutgerinnungsfaktors VII umfasst,
wobei das Derivat des Blutgerinnungsfaktors VII durch Bindung eines Peptidlinkers, der Cystein als letzten Aminosäurerest an seinem C-Terminus aufweist, an den C-Terminus von Blutgerinnungsfaktor VII hergestellt wird und wobei der Gehalt an aktiviertem Blutgerinnungsfaktor-VII-Derivat in der Zusammensetzung weniger als 5 %, bezogen auf das Blutgerinnungsfaktor-VII-Derivat insgesamt, beträgt,
wobei das Blutgerinnungsfaktor-VII-Derivat insgesamt sowohl aktivierte als auch inaktivierte Formen umfasst.

2. Verfahren nach Anspruch 1, wobei es sich bei der Zusammensetzung um eine flüssige Zusammensetzung handelt.

3. Verfahren nach Anspruch 2, wobei die flüssige Zusammensetzung einen pH 5,0 bis 8,0 aufweist.

4. Verfahren nach Anspruch 2, wobei die Zusammensetzung eine Temperatur von 4 bis 25 °C aufweist.

5. Verfahren nach Anspruch 1, wobei die Konzentration des Blutgerinnungsfaktor-VII-Derivats in der Zusammensetzung 0,01 mg/ml bis 5,0 mg/ml beträgt.

6. Verfahren nach Anspruch 1, wobei das Tensid aus der aus Tween, Polysorbat 20, Polysorbat 60, Polysorbat 80, Ploxamer 188, Triton X-100 und Kombinationen davon bestehenden Gruppe ausgewählt ist.

7. Verfahren nach Anspruch 1, wobei das Tensid in einer Endkonzentration von 0,01 bis 1,00 % (Gew./Vol.) zugesetzt wird.

8. Verfahren nach Anspruch 7, wobei das Tensid in einer Endkonzentration von 0,1 bis 0,5 % (Gew./Vol.) zugesetzt wird.

9. Verfahren nach Anspruch 1, wobei der Peptidlinker eine Peptidsequenz aufweist, die aus der aus SEQ ID NO: 3 und 6 bis 12 bestehenden Gruppe ausgewählt ist.

## Revendications

1. Procédé d'inactivation de virus, comprenant l'ajout d'un tensioactif à une composition comprenant un dérivé de facteur VII de coagulation sanguine,
dans lequel le dérivé de facteur VII de coagulation sanguine est préparé en liant un lieur peptidique contenant de la cystéine en tant que résidu d'acide aminé final de l'extrémité C-terminale de celui-ci à l'extrémité C-terminale du facteur VII de coagulation sanguine, et dans lequel la teneur du dérivé de facteur VII de coagulation sanguine activé dans la composition est inférieure à 5 %, sur la base du dérivé de facteur VII de coagulation sanguine total,
dans lequel le dérivé de facteur VII de coagulation sanguine totale comprend à la fois des formes activées et inactivées.

2. Procédé selon la revendication 1, dans lequel la composition est une composition liquide.

3. Procédé selon la revendication 2, dans lequel la composition liquide a un pH de 5,0 à 8,0.

4. Procédé selon la revendication 2, dans lequel la composition a une température de 4 à 25°C.

5. Procédé selon la revendication 1, dans lequel la concentration du dérivé de facteur VII de coagulation sanguine dans la composition est de 0,01 mg/mL à 5,0 mg/mL.

6. Procédé selon la revendication 1, dans lequel le tensioactif est choisi dans le groupe comprenant le Tween, le polysorbate 20, le polysorbate 60, le polysorbate 80, le ploxamère 188, le Triton X-100, et des combinaisons de ceux-ci.

7. Procédé selon la revendication 1, dans lequel le tensioactif est ajouté à une concentration finale de 0,01 à 1,00 % (poids/volume).

8. Procédé selon la revendication 7, dans lequel le tensioactif est ajouté à une concentration finale de 0,1 à 0,5 % (poids/volume).

9. Procédé selon la revendication 1, dans lequel le lieur peptidique a une séquence peptidique choisie dans le groupe constitué des SEQ ID NO: 3 et 6 à 12.


<110> HANMI PHARM. CO., LTD.
<120> A method of virus inactivation in composition comprising Factor VII
<130> OPA13189/PCT
<150> KR 10-2013-0011472
   <151> 2013-01-31
<160> 20
<170> KopatentIn 2.0
<210> 1
   <211> 34
   <212> DNA
   <213> Artificial Sequence
<220>
   <223> primer
<400> 1
   cccggatcca tggtctccca ggccctcagg tcc 34
<210> 2
   <211> 31
   <212> DNA
   <213> Artificial Sequence
<220>
   <223> primer
<400> 2
   gggctcgagc tagggaaatg gggctcgcag g 31
<210> 3
   <211> 6
   <212> PRT
   <213> Artificial Sequence
<220>
   <223> peptide linker
<400> 3
   Ala Thr Lys Ala Val Cys
   1 5
<210> 4
   <211> 45
   <212> DNA
   <213> Artificial Sequence
<220>
   <223> primer
<400> 4
   ccggaattca tggccaacgc gttcctggag gagctgcggc cgggc 45
<210> 5
   <211> 62
   <212> DNA
   <213> Artificial Sequence
<220>
   <223> primer
<400> 5
   ccgctcgagt cagcacacgg ccttcgtcgc gggaaatggg gctcgcagga ggactcctgg 60
   gc 62
<210> 6
   <211> 6
   <212> PRT
   <213> Artificial Sequence
<220>
   <223> peptide linker
<400> 6
   Gly Gly Gly Gly Ser Cys
   1 5
<210> 7
   <211> 149
   <212> PRT
   <213> Artificial Sequence
<220>
   <223> Human SOD1 1-149
<400> 7
   Ala Thr Lys Ala Val Cys Val Leu Lys Gly Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp Lys Asp Gly Val Ala Asp
                   85                  90                  95
   Val Ser Ile Glu Asp Ser Val Ile Ser Leu Ser Gly Asp His Cys Ile
               100                 105                 110
   Ile Gly Arg Thr Leu Val Val His Glu Lys Ala Asp Asp Leu Gly Lys
           115                 120                 125
   Gly Gly Asn Glu Glu Ser Thr Lys Thr Gly Asn Ala Gly Ser Arg Leu
       130                 135                 140
   Ala Cys Gly Val Ile
   145
<210> 8
   <211> 149
   <212> PRT
   <213> Artificial Sequence
<220>
   <223> modified Human SOD1 1-149
<400> 8
   Ala Thr Lys Ala Val Cys Ile Pro Arg Ile Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp Lys Asp Gly Val Ala Asp
                   85                  90                  95
   Val Ser Ile Glu Asp Ser Val Ile Ser Leu Ser Gly Asp His Cys Ile
               100                 105                 110
   Ile Gly Arg Thr Leu Val Val His Glu Lys Ala Asp Asp Leu Gly Lys
           115                 120                 125
   Gly Gly Asn Glu Glu Ser Thr Lys Thr Gly Asn Ala Gly Ser Arg Leu
       130                 135                 140
   Ala Cys Gly Val Ile
   145
<210> 9
   <211> 90
   <212> PRT
   <213> Artificial Sequence
<220>
   <223> Human SOD1 1-90
<400> 9
   Ala Thr Lys Ala Val Cys Val Leu Lys Gly Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp
                   85                  90
<210> 10
   <211> 90
   <212> PRT
   <213> Artificial Sequence
<220>
   <223> modified Human SOD1 1-90
<400> 10
   Ala Thr Lys Ala Val Cys Ile Pro Arg Ile Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp
                   85                  90
<210> 11
   <211> 25
   <212> PRT
   <213> Artificial Sequence
<220>
   <223> Human SOD1 1-25
<400> 11
   Ala Thr Lys Ala Val Cys Val Leu Lys Gly Asp Gly Pro Val Gln Gly
   1               5                   10                  15
   Ile Ile Asn Phe Glu Gln Lys Glu Ser
               20                  25
<210> 12
   <211> 25
   <212> PRT
   <213> Artificial Sequence
<220>
   <223> modified Human SOD1 1-25
<400> 12
   Ala Thr Lys Ala Val Cys Ile Pro Arg Ile Asp Gly Pro Val Gln Gly
   1               5                   10                  15
   Ile Ile Asn Phe Glu Gln Lys Glu Ser
               20                  25
<210> 13
   <211> 450
   <212> PRT
   <213> Artificial Sequence
<220>
   <223> FactorVII-ATKAVC
<400> 13
   Met Val Ser Gln Ala Leu Arg Leu Leu Cys Leu Leu Leu Gly Leu Gln
   1               5                   10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys
       450
<210> 14
   <211> 450
   <212> PRT
   <213> Artificial Sequence
<220>
   <223> FactorVII-GGGGSC
<400> 14
   Met Val Ser Gln Ala Leu Arg Leu Leu Cys Leu Leu Leu Gly Leu Gln
   1               5                   10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
   340 345 350
   Arg Lys Val Gly Asp Ser Pro Asn Ile Thr Glu Tyr Met Phe Cys Ala
   355 360 365
   Gly Tyr Ser Asp Gly Ser Lys Asp Ser Cys Lys Gly Asp Ser Gly Gly
   370 375 380
   Pro His Ala Thr His Tyr Arg Gly Thr Trp Tyr Leu Thr Gly Ile Val
   385 390 395 400
   Ser Trp Gly Gln Gly Cys Ala Thr Val Gly His Phe Gly Val Tyr Thr
   405 410 415
   Arg Val Ser Gln Tyr Ile Glu Trp Leu Gln Lys Leu Met Arg Ser Glu
   420 425 430
   Pro Arg Pro Gly Val Leu Leu Arg Ala Pro Phe Pro Gly Gly Gly Gly
   435 440 445
   Ser Cys
   450
<210> 15
   <211> 593
   <212> PRT
   <213> Artificial Sequence
<220>
   <223> FactorVII-SOD1 1^{∼}149
<400> 15
   Met Val Ser Gln Ala Leu Arg Leu Leu Cys Leu Leu Leu Gly Leu Gln
   1               5                   10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys Val Leu Lys Gly Asp Gly Pro Val Gln Gly Ile Ile Asn Phe
       450                 455                 460
   Glu Gln Lys Glu Ser Asn Gly Pro Val Lys Val Trp Gly Ser Ile Lys
   465                 470                 475                 480
   Gly Leu Thr Glu Gly Leu His Gly Phe His Val His Glu Phe Gly Asp
                   485                 490                 495
   Asn Thr Ala Gly Cys Thr Ser Ala Gly Pro His Phe Asn Pro Leu Ser
               500                 505                 510
   Arg Lys His Gly Gly Pro Lys Asp Glu Glu Arg His Val Gly Asp Leu
           515                 520                 525
   Gly Asn Val Thr Ala Asp Lys Asp Gly Val Ala Asp Val Ser Ile Glu
       530                 535                 540
   Asp Ser Val Ile Ser Leu Ser Gly Asp His Cys Ile Ile Gly Arg Thr
   545                 550                 555                 560
   Leu Val Val His Glu Lys Ala Asp Asp Leu Gly Lys Gly Gly Asn Glu
                   565                 570                 575
   Glu Ser Thr Lys Thr Gly Asn Ala Gly Ser Arg Leu Ala Cys Gly Val
               580                 585                 590
   Ile
<210> 16
   <211> 593
   <212> PRT
   <213> Artificial Sequence
<220>
   <223> FactorVII-SOD1 IPRI
<400> 16
   Met Val Ser Gln Ala Leu Arg Leu Leu Cys Leu Leu Leu Gly Leu Gln
   1               5                   10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys Ile Pro Arg Ile Asp Gly Pro Val Gln Gly Ile Ile Asn Phe
       450                 455                 460
   Glu Gln Lys Glu Ser Asn Gly Pro Val Lys Val Trp Gly Ser Ile Lys
   465                 470                 475                 480
   Gly Leu Thr Glu Gly Leu His Gly Phe His Val His Glu Phe Gly Asp
                   485                 490                 495
   Asn Thr Ala Gly Cys Thr Ser Ala Gly Pro His Phe Asn Pro Leu Ser
   500 505 510
   Arg Lys His Gly Gly Pro Lys Asp Glu Glu Arg His Val Gly Asp Leu
   515 520 525
   Gly Asn Val Thr Ala Asp Lys Asp Gly Val Ala Asp Val Ser Ile Glu
   530 535 540
   Asp Ser Val Ile Ser Leu Ser Gly Asp His Cys Ile Ile Gly Arg Thr
   545 550 555 560
   Leu Val Val His Glu Lys Ala Asp Asp Leu Gly Lys Gly Gly Asn Glu
   565 570 575
   Glu Ser Thr Lys Thr Gly Asn Ala Gly Ser Arg Leu Ala Cys Gly Val
   580 585 590
   Ile
<210> 17
   <211> 534
   <212> PRT
   <213> Artificial Sequence
<220>
   <223> FVII-SOD1 1-90
<400> 17
   Met Val Ser Gln Ala Leu Arg Leu Leu Cys Leu Leu Leu Gly Leu Gln
   1 5 10 15
   Gly Cys Leu Ala Ala Val Phe Val Thr Gln Glu Glu Ala His Gly Val
   20 25 30
   Leu His Arg Arg Arg Arg Ala Asn Ala Phe Leu Glu Glu Leu Arg Pro
   35 40 45
   Gly Ser Leu Glu Arg Glu Cys Lys Glu Glu Gln Cys Ser Phe Glu Glu
   50 55 60
   Ala Arg Glu Ile Phe Lys Asp Ala Glu Arg Thr Lys Leu Phe Trp Ile
   65 70 75 80
   Ser Tyr Ser Asp Gly Asp Gln Cys Ala Ser Ser Pro Cys Gln Asn Gly
   85 90 95
   Gly Ser Cys Lys Asp Gln Leu Gln Ser Tyr Ile Cys Phe Cys Leu Pro
   100 105 110
   Ala Phe Glu Gly Arg Asn Cys Glu Thr His Lys Asp Asp Gln Leu Ile
   115 120 125
   Cys Val Asn Glu Asn Gly Gly Cys Glu Gln Tyr Cys Ser Asp His Thr
   130 135 140
   Gly Thr Lys Arg Ser Cys Arg Cys His Glu Gly Tyr Ser Leu Leu Ala
   145 150 155 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys Val Leu Lys Gly Asp Gly Pro Val Gln Gly Ile Ile Asn Phe
       450                 455                 460
   Glu Gln Lys Glu Ser Asn Gly Pro Val Lys Val Trp Gly Ser Ile Lys
   465                 470                 475                 480
   Gly Leu Thr Glu Gly Leu His Gly Phe His Val His Glu Phe Gly Asp
                   485                 490                 495
   Asn Thr Ala Gly Cys Thr Ser Ala Gly Pro His Phe Asn Pro Leu Ser
               500                 505                 510
   Arg Lys His Gly Gly Pro Lys Asp Glu Glu Arg His Val Gly Asp Leu
           515                 520                 525
   Gly Asn Val Thr Ala Asp
       530
<210> 18
   <211> 534
   <212> PRT
   <213> Artificial Sequence
<220>
   <223> FVII-SOD1 1-90 IPRI
<400> 18
   Met Val Ser Gln Ala Leu Arg Leu Leu Cys Leu Leu Leu Gly Leu Gln
   1               5                   10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys Ile Pro Arg Ile Asp Gly Pro Val Gln Gly Ile Ile Asn Phe
       450                 455                 460
   Glu Gln Lys Glu Ser Asn Gly Pro Val Lys Val Trp Gly Ser Ile Lys
   465                 470                 475                 480
   Gly Leu Thr Glu Gly Leu His Gly Phe His Val His Glu Phe Gly Asp
                   485                 490                 495
   Asn Thr Ala Gly Cys Thr Ser Ala Gly Pro His Phe Asn Pro Leu Ser
               500                 505                 510
   Arg Lys His Gly Gly Pro Lys Asp Glu Glu Arg His Val Gly Asp Leu
           515                 520                 525
   Gly Asn Val Thr Ala Asp
       530
<210> 19
   <211> 469
   <212> PRT
   <213> Artificial Sequence
<220>
   <223> FVII-SOD1 1-25
<400> 19
   Met Val Ser Gln Ala Leu Arg Leu Leu Cys Leu Leu Leu Gly Leu Gln
    1               5                  10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305 310 315 320
   Gly Trp Gly Gln Leu Leu Asp Arg Gly Ala Thr Ala Leu Glu Leu Met
   325 330 335
   Val Leu Asn Val Pro Arg Leu Met Thr Gln Asp Cys Leu Gln Gln Ser
   340 345 350
   Arg Lys Val Gly Asp Ser Pro Asn Ile Thr Glu Tyr Met Phe Cys Ala
   355 360 365
   Gly Tyr Ser Asp Gly Ser Lys Asp Ser Cys Lys Gly Asp Ser Gly Gly
   370 375 380
   Pro His Ala Thr His Tyr Arg Gly Thr Trp Tyr Leu Thr Gly Ile Val
   385 390 395 400
   Ser Trp Gly Gln Gly Cys Ala Thr Val Gly His Phe Gly Val Tyr Thr
   405 410 415
   Arg Val Ser Gln Tyr Ile Glu Trp Leu Gln Lys Leu Met Arg Ser Glu
   420 425 430
   Pro Arg Pro Gly Val Leu Leu Arg Ala Pro Phe Pro Ala Thr Lys Ala
   435 440 445
   Val Cys Val Leu Lys Gly Asp Gly Pro Val Gln Gly Ile Ile Asn Phe
   450 455 460
   Glu Gln Lys Glu Ser
   465
<210> 20
   <211> 469
   <212> PRT
   <213> Artificial Sequence
<220>
   <223> FactorVII-SOD1 1^{∼}25 IPRI
<400> 20
   Met Val Ser Gln Ala Leu Arg Leu Leu Cys Leu Leu Leu Gly Leu Gln
   1 5 10 15
   Gly Cys Leu Ala Ala Val Phe Val Thr Gln Glu Glu Ala His Gly Val
   20 25 30
   Leu His Arg Arg Arg Arg Ala Asn Ala Phe Leu Glu Glu Leu Arg Pro
   35 40 45
   Gly Ser Leu Glu Arg Glu Cys Lys Glu Glu Gln Cys Ser Phe Glu Glu
   50 55 60
   Ala Arg Glu Ile Phe Lys Asp Ala Glu Arg Thr Lys Leu Phe Trp Ile
   65 70 75 80
   Ser Tyr Ser Asp Gly Asp Gln Cys Ala Ser Ser Pro Cys Gln Asn Gly
   85 90 95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys Ile Pro Arg Ile Asp Gly Pro Val Gln Gly Ile Ile Asn Phe
       450                 455                 460
   Glu Gln Lys Glu Ser
   465